(19) Europäisches Patentamt
European Patent Office
Office européen des brevets

(11) Publication number: **0 007 206**
**B1**

(12) # EUROPEAN PATENT SPECIFICATION

(45) Date of publication of patent specification: **23.02.83**

(21) Application number: **79301282.4**

(22) Date of filing: **02.07.79**

(51) Int. Cl.$^3$: **C 07 C 91/30,** A 61 K 31/135, A 61 K 31/165, A 61 K 31/18, C 07 C 103/28, C 07 C 103/44, C 07 C 103/76, C 07 C 143/74

(54) **Phenethanolamines, their formulations, preparation and use.**

(30) Priority: **03.07.78 US 921668**

(43) Date of publication of application:
**23.01.80 Bulletin 80/2**

(45) Publication of the grant of the patent:
**23.02.83 Bulletin 83/8**

(84) Designated Contracting States:
**DE GB IT NL SE**

(56) References cited:
**DE - A - 2 048 555**
**US - A - 3 801 631**

(73) Proprietor: **ELI LILLY AND COMPANY**
**307, East McCarty Street**
**Indianapolis Indiana 46285 (US)**

(72) Inventor: **Mills, Jack**
**7902, Timberhill Drive**
**Indianapolis, Indiana 46217 (US)**
Inventor: **Schmiegel, Klaus Kurt**
**4507 Staughton Drive**
**Indianapolis, Indiana 46226 (US)**
Inventor: **Toomey, Richard Eugene**
**11425 Lakeshore Drive West**
**Carmel, Indiana 46032 (US)**

(74) Representative: **Crowther, Terence Roger**
**European Patent Attorney et al,**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 007 206 B1

# Phenethanolamines, their formulations, preparations and use

This invention relates to novel phenethanolamines useful in potentiating the antineoplastic effects of oncolytic drugs, to pharmaceutical formulations containing the novel phenethanolamine derivatives either alone or in combination with known oncolytic agents and to their preparation.

Whilst a great number of compounds have been investigated for their usefulness in the treatment of neoplastic diseases, relatively few have proven sufficiently useful in the clinical treatment of human neoplasms. The major drawback in the use of all known oncolytic drugs is their levels of toxicity. It has been established, for example, that the use of certain antineoplastic drugs at those levels required to clinically treat or eradicate a tumor can cause later growth of a different neoplasm. In an effort to combat the extreme toxic effects of continued doses of various antitumor drugs, combination chemotherapy has been utilized. By employing more than one antineoplastic drug, a decrease in the required dosage of each component drug, and consequently the accumulated toxic effects attributable to each individual drug, can be realized. In all such combination chemotherapy, all of the component drugs heretofore have been clinically effective antineoplastic agents, and all have had their own toxic effects.

The compounds of this invention are unique in that they do not possess antineoplastic activity themselves, at contemplated dose levels, yet are capable of increasing the beneficial effects of drugs known to be clinically useful antineoplastic agents.

Compounds similar to those of the present invention have been described in U.S. Patent Specification No. 3,816,516 as $\beta$-agonists useful in the chemotherapy of heart disease.

This invention provides compounds having the general formula (I):

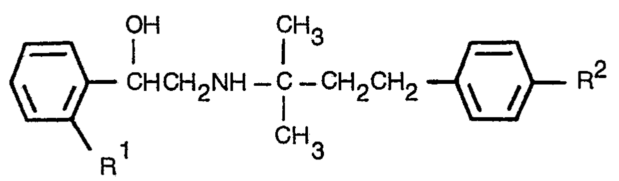

(I)

wherein:

$R^1$ is hydrogen or fluorine;

$R^2$ is hydroxy, aminocarbonyl, methylaminocarbonyl, dimethylaminocarbonyl, acetylamino or methanesulfonylamino;

and the pharmaceutically acceptable acid addition salts thereof.

The compounds of this invention are useful in potentiating the antineoplastic activity of known oncolytic drugs. Such known drugs include agents such as cytoxan; antimetabolites such as 5-fluorouracil, and 6-mercaptopurine; as well as plant alkaloids such as vindesine, vincristine and vinblastine. Methotrexate also is potentiated by the claimed compounds. The compounds of this invention preferably are used to potentiate the oncolytic activity of agents such as cytoxan, 5-fluorouracil, 6-mercaptopurine, methotrexate, and the vinca alkaloids. Tumors which can be effectively treated with oncolytic drugs which are potentiated by a compound of this invention include solid tumors, particularly neuroblastoma, and solid tumors of the breast, ovary, lung, colon, stomach, pancreas and urinary tract. Additionally treated are tumors such as testicular carcinoma, adenocarcinomas, and retinoblastoma. The invention will be practiced by administering a potentiating amount of a compound of this invention to a subject receiving an antineoplastic drug known to be useful in the particular disease being treated. The effectiveness of such known oncolytic drug is significantly increased by the action of a compound of this invention so that the desired clinical effects of the oncolytic drug are realized.

The present invention therefore provides compounds of formula I, stated above, or pharmaceutically acceptable acid addition salts thereof, for use in the potentiation of the oncolytic effect of an antineoplastic agent.

In another aspect of the invention there is provided a pharmaceutical formulation which comprises a compound of formula (I), or a pharmaceutically-acceptable acid addition salt thereof, associated with a pharmaceutically-acceptable carrier therefor. The active component of the formulation may also include a known oncolytic agent.

The compounds of the invention may be employed in methods for treating neoplastic diseases in mammals comprising the administration of a clinically effective or suboptimal dose of an oncolytic drug known to be effective against the particular neoplastic disease being treated, together with a potentiating amount of a compound of this invention. The compounds provided herein preferably are used to potentiate the antineoplastic activity of a compound selected from cytoxan, 5-fluorouracil, methotrexate, 6-mercaptopurine and the vinca alkaloid oncolytic drugs. Treatment of solid tumors in humans may be effected by administering a compound of this invention and a drug known to be effective in the treatment of such solid tumors.

The compounds provided by this invention can be prepared by any of a number of methods utilizing well known principles of organic chemistry. One such method of preparation includes the

alkylation of a 1,1-dimethyl-3-arylpropylamine with a haloacetophenone, i.e. a benzoylmethyl halide. Such alkylation provides an N-benzoylmethyl-1,1-dimethyl-3-arylpropylamine. The benzoyl carbonyl moiety is then reduced to provide a compound of the invention.

In other words, a carbonyl compound of formula (II):

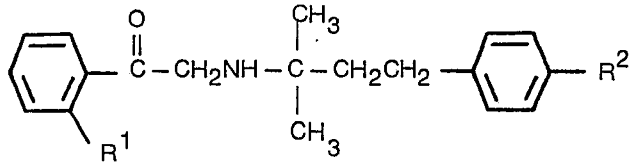

(II)

is reduced. The compounds of formula (II) are novel and provided in one aspect of the invention is the single stage process for producing compounds of formula (I) by reduction of compounds of formula (II). The reduction is preferably effected within the temperature range from 15 to 115°C. in an inert organic solvent.

As an illustration of such generalized synthetic process, a benzoylmethyl halide such as benzoylmethyl bromide, *o*-fluorobenzoylmethyl iodide, or the like, is reacted with a propylamine derivative such as 1,1-dimethyl-3-(4-hydroxyphenyl)propylamine. It is often preferred that the phenolic hydroxyl group of such arylpropylamine be protected during such condensation reactions, thereby obviating the possibility of unwanted side reactions. Commonly used hydroxyl protecting groups include ether forming groups such as methyl and benzyl, as well as esters such as acetate and the like. The benzoylmethyl halide and the 1,1-dimethyl-3-arylpropylamine generally are utilized in approximately equimolar quantities; however, an excess of the amine can be utilized if desired and then later separated from the desired product by routine methods. The alkylation reaction is best carried out in a suitable solvent such as diethyl ether, benzene, dichloromethane, or the like, and normally is complete within about two to ten hours when carried out at about 25 to about 70°C. The N-benzoylmethyl-1,1-dimethyl-3-arylpropylamine which is formed normally is recovered simply by filtering the reaction mixture, thereby removing any unreacted starting materials, and then removing the solvent from the filtrate, for instance by evaporation. The product thus formed can be further purified if desired by routine methods such as salt formation and crystallization. The N-benzoylmethyl-propylamine derivative next is reduced to provide a compound of this invention. Such reduction is accomplished by any of the commonly used methods to reduce a carbonyl group to a carbinol moiety, including catalytic hydrogenation utilizing catalysts such as platinum or palladium, or metal hydride reductions utilizing agents such as sodium borohydride and the like.

An alternative method for preparing the compounds provided by this invention comprises the coupling of a mandelic acid or mandelic acid derivative with a 1,1-dimethyl-3-arylpropylamine to form the corresponding amide, and then reducing the amide carbonyl moiety to a methylene group. This method of preparation normally is not utilized for preparing compounds having an amido substituent at the 4-position of the 3-phenylpropyl moiety (i.e. $R^2$ is an amido group), since some reduction of such amido group is likely to result.

The direct coupling of an amine and a mandelic acid can be accomplished in several recognized ways. For instance, a mandelic acid ester or anhydride can be condensed with an amine such as 1,1-dimethyl-3-(4-hydroxyphenyl)propylamine to form the corresponding amide. Alternatively, a mandelic acid as the free acid can be reacted with an amine in the presence of any of the commonly used peptide coupling reagents. Such coupling reagents include N,N′-diisopropylcarbodiimide, N,N′-dicyclohexylcarbodiimide, carbonyldiimidazole, N-ethoxycarbonyl-2-ethoxy-1,2-dihydroquinoline and the like. The coupling reaction generally is conducted in an organic solvent such as benzene, dichloromethane, dimethylformamide, hexamethylphosphoramide, and related solvents, and normally is complete within about two to twenty-four hours when carried out at a temperature of about −10 to about 50°C. When the reaction is complete, the product is readily recovered by simply filtering the reaction mixture and removing the reaction solvent. The product thus formed can be purified if desired by standard methods such as chromatography, crystallization and the like.

The coupling of a mandelic acid with a 1,1-dimethyl-3-arylpropylamine utilizing a peptide coupling agent is a particularly preferred synthetic route when it is desired to prepare an optically active compound of this invention. It should be noted that the S-optical isomers of the above formula are substantially devoid of biological activity, most or all of the useful activity residing in the R-optical isomers. The R-optical isomers of the above formula are therefore the important compounds of this invention. It should be further noted, however, that since the inactive S-isomers do not impart toxic effects to a biological system at the dose levels contemplated, that it may be desirable from an economic standpoint to utilize the racemic dl mixture of isomers, thereby obviating the need to separate isomers or secure optically active starting materials.

If it is desired to prepare an optically active R-isomer of the above formula, a preferred method for doing so comprises reacting an optically active mandelic acid such as R-*o*-fluoromandelic acid with an amine such as 1,1-dimethyl-3-(4-hydroxyphenyl)propylamine in the presence of a coupling reagent

such as N,N'-dicyclohexylcarbodiimide. If desired, an agent to inhibit racemization can be utilized in the coupling reactions. A commonly used catalyst and anti-racemization agent is 1-hydroxybenzotriazole.

The amides which are formed by the above described coupling reactions of a mandelic acid and an amine are next subjected to a reduction reaction to transform the amide carbonyl to a methylene group, thereby providing a compound of this invention. Typical reducing agents commonly used to effect the desired reduction of the amide carbonyl include metal hydrides such as lithium aluminium hydride, diisobutyl aluminium hydride, as well as diborane. The reductions generally are carried out in unreactive organic solvents such as diethyl ether, tetrahydrofuran, benzene, and the like, and routinely are complete within about two to twenty hours when conducted at about 30°C. If desired, a solubilizing agent such as trimethylchlorosilane can be added to the reaction to facilitate solubilization of the amide to be reduced. As an illustration of such reductions, an amide such as R-N-[2-(2-fluorophenyl)-2-hydroxy-1-oxoethyl]-1,1-dimethyl-3-(4-hydroxyphenyl)propylamine can be reacted with about a two molar excess of diborane in tetrahydrofuran to provide the corresponding amine, namely R-N-[2-(2-fluorophenyl)-2-hydroxyethyl]-1,1-dimethyl-3-(4-hydroxyphenyl)propylamine. The amine so formed can be further purified by crystallization, salt formation, and related techniques.

Another and often preferred method for preparing all of the compounds comprehended by this invention comprises reacting a 1,1-dimethyl-3-(4-substituted phenyl)propylamine with styrene oxide, *o*-fluorostyrene oxide, or even an optically active styrene oxide. The amine and styrene oxide are utilized in approximately equimolar quantities, and the reaction is carried out in a polar organic solvent such as methanol or ethanol. The condensation to provide directly a compound of this invention generally is complete within about ten to twenty hours when the reaction is carried out at about 50 to about 80°C., although temperatures from 20 to 150°C. can be utilized. The product, a compound of this invention, is isolated by simply removing the reaction solvent, for instance by evaporation under reduced pressure. The product amine can be further purified if desired by crystallization from solvents such as acetone or ethyl acetate, or alternatively can be converted to a salt and crystallized from solvents such as methanol or isopropanol.

As can be seen from the above general formula, the compounds of this invention are secondary amines, and since the amine nitrogen atom is basic in nature, the compounds readily form acid addition salts with any of a number of inorganic and organic acids. Pharmaceutically acceptable acid addition salts of amines having the above formula are therefore provided by this invention. While the particular acid utilized to form the pharmaceutically acceptable salt is not critical, the resulting salt should have no undesirable pharmacological properties not possessed by the parent amine base. Any of a wide variety of inorganic acids can be utilized to form the non-toxic pharmaceutically acceptable salts of this invention, and those most commonly used include hydrochloric acid, sulfuric acid, perchloric acid, phosphoric acid, nitric acid and the like. Commonly used organic acids include acetic acid, butyric acid, maleic acid, succinic acid, lactic acid, oxalic acid, *p*-toluenesulfonic acid, and related acids.

Like most amine acid addition salts, the non-toxic pharmaceutically acceptable acid addition salts comprehended by this invention are characteristically highly crystalline solids and thus lend themselves to ready purification by crystallization from common solvents. Such salts can, of course, be converted back to the amine free base simply by reaction with a base such as sodium hydroxide or potassium carbonate. Because the salts of this invention are highly crystalline and water soluble, it often is desirable to utilize the salts when formulating a drug for oral or parenteral administration.

Representative of the compounds comprehended and provided by this invention are as follows:

N - (2 - phenyl - 2 - hydroxyethyl) - 1,1 - dimethyl - 3 - (4 - dimethylaminocarbonylphenyl)propylamine;

R - N - (2 - phenyl - 2 - hydroxyethyl) - 1,1 - dimethyl - 3 - (4 - aminocarbonylphenyl)-propylamine;

N - [2 - (2 - fluorophenyl) - 2 - hydroxyethyl] - 1,1 - dimethyl - 3 - (4 - hydroxyphenyl)-propylamine;

N - [2 - (2 - fluorophenyl) - 2 - hydroxyethyl] - 1,1 - dimethyl - 3 - (4 - methylaminocarbonylphenyl)propylaminium bromide;

N - (2 - phenyl - 2 - hydroxyethyl) - 1,1 - dimethyl - 3 - (4 - hydroxyphenyl)propylaminium acetate;

R - N - (2 - phenyl - 2 - hydroxyethyl) - 1,1 - dimethyl - 3 - (4 - methylaminocarbonylphenyl)propylaminium isobutyrate;

R - N - [2 - (2 - fluorophenyl) - 2 - hydroxyethyl] - 1,1 - dimethyl - 3 - (4 - hydroxyphenyl)propylaminium chloride;

R - N - [2 - (2 - fluorophenyl) - 2 - hydroxyethyl] - 1,1 - dimethyl - 3 - (4 - dimethylaminocarbonylphenyl)propylaminium oxalate;

R - N - (2 - phenyl - 2 - hydroxyethyl) - 1,1 - dimethyl - 3 - (4 - aminocarbonylphenyl)-propylaminium *p*-toluenesulfonate; and the like.

As hereinbefore pointed out, the compounds of this invention are useful as potentiators of known and currently used antineoplastic drugs. In other words, the compounds of this invention cause a

significant and unexpected increase in the effectiveness of currently used oncolytic agents. Such surprising result permits the use of now clinically used antineoplastic drugs in lesser amounts than now permitted for effective treatment, or permits more effective treatment when administering oncolytic drugs at their current clinical levels. Since the compounds of this invention are neither antineoplastic in themselves nor toxic to healthy cells at contemplated doses, the overall effect of combination chemotherapy according to this invention is that greater efficacy and fewer toxic side effects from the use of known oncolytic drugs can be realized in the treatment of tumors.

The potentiating activity of the compounds of this invention has been demonstrated in a number of laboratory tests. One such test, for example, was a determination of mitotic inhibitory activity. The compounds of this invention have the ability to arrest cultured cells at the mitotic phase (metaphase) of the cell cycle without apparent effect on other stages of the cell cycle. Such mitotic inhibitory activity is an indication that the compounds may have a beneficial effect on the biological activities of antineoplastic drugs. Such mitotic inhibitory activity was demonstrated in Chinese hamster ovary cells. The compounds of the invention were administered at 20 mcg./ml. and the number of cells arrested at the mitotic phase out of 1000 cultured cells were counted and compared to the controls which underwent mitotic arrest without a drug present. Table I presents the results of the mitotic arrest assay. Two evaluations were carried out for each test compound. The results reported are the percent of cells held at mitosis after five hours contact with the compound of this invention.

Table I

Mitotic Index (controls average = percent in the mitotic phase)

| | 20.0 mcg/ml |
|---|---|
| N-(2-phenyl-2-hydroxyethyl)-1,1-dimethyl-3-(4-hydroxyphenyl)propylamine | 36<br>33 |
| N-(2-phenyl-2-hydroxyethyl)-ethyl)-1,1-dimethyl-3-(4-aminocarbonylphenyl)propylamine | 57<br>45 |
| N-(2-phenyl-2-hydroxyethyl)-1,1-dimethyl-3-(4-dimethylaminocarbonylphenyl)propylamine | 40<br>46 |
| N-(2-phenyl-2-hydroxyethyl)-1,1-dimethyl-3-(4-methylaminocarbonylphenyl)propylamine | 36<br>38 |

The compounds provided by this invention have been shown to significantly increase the efficacy of antineoplastic agents when administered to mice and hamsters suffering from implanted tumors. In a typical experiment, laboratory mice were infected by a subcutaneous implantation of a tumor tissue in the axillary region of the body. The implantations were accomplished by means of a trocar. Initial tumor size in each animal generally was about 2 square millimeters. Tumor size was then measured periodically as length and width in mm. Typical tumor systems implanted included adenocarcinoma 755, an Aly Fast strain of bronchiogenic carcinoma, and mecca lymphosarcoma. Oncolytic agents utilized in the tests to determine the potentiating effects of the compounds of the invention include cytoxan, 5-fluorouracil, vindesine, methotrexate and 6-mercaptopurine. Twenty or thirty infected animals generally were selected as controls and received placebo. Ten infected animals normally were selected to receive a known oncolytic agent. Another ten infected animals were administered the known oncolytic agent in addition to a compound of this invention.

Table 2 presents the results of combination chemotherapy in the treatment of mice infected with an adenocarcinoma tumor resembling human breast tumor (the 755 tumor system). The table shows the results of treatment for twenty-eight days with placebo, with 6-mercaptopurine alone (designated in the Table as 6 MP), and with the combination of 6 MP plus a compound of this invention, N-(2-phenyl-2-hydroxyethyl)-1,1-dimethyl-3-(4-hydroxyphenyl)propylaminium bromide (designated in the Table as A). According to the test, 6 MP was administered alone to 10 mice orally at the dose of 1.5 mg./kg. once each day for twenty-eight days. Another group of ten mice received 6 MP daily (1.5 mg./kg.) plus the compound of this invention (A), which was administered subcutaneously at the rate of 3.75 mg./kg. twice daily for twenty-eight days. The results of treatment are reported as the average size of tumor growth, given in square millimeters, and the number of surviving test animals out of those undergoing treatment in each test group. Measurement of tumor sizes were made on the days following the initial tumor implantation as indicated in the table. The average tumor size for the test

group on the indicated day of measurement is reported in column I under each of the reported days of measurement. In column II of each of the reported days of measurement is recorded the number of surviving animals out of the particular test group.

Table 2

| Compound administered | Day 0 | | 9 | | 17 | | 23 | | 28 | |
|---|---|---|---|---|---|---|---|---|---|---|
| | I | II | I | II | I | II | I | II | I | II |
| controls | — | 20/20 | 240 | 20/20 | 850 | 15/20 | — | 0/20 | — | 0/20 |
| 6 MP | — | 10/10 | 100 | 9/10 | 340 | 7/10 | 680 | 6/10 | 1050 | 1/10 |
| 6 MP + A | — | 10/10 | 75 | 10/10 | 75 | 10/10 | 45 | 9/10 | 40 | 9/10 |

A similar study was carried out to determine the synergistic effect of N-(2-phenyl-2-hydroxyethyl)-1,1-dimethyl-3-(4-hydroxyphenyl)propylaminium bromide (compound A) on the antineoplastic activity of 5-fluorouracil (designated in Table 3 as 5 FU) in the treatment of a meccalymphosarcoma in mice. 5-Fluorouracil was administered intraperitoneally at the rate of 4 mg./kg. once each day to a group of five test animals. Another group of five infected animals received the combination of 4 mg./kg. of 5 FU given intraperitoneally once each day plus the potentiating agent (compound A) given subcutaneously twice each day at the dose of 18.75 mg./kg. A group of 20 infected animals received placebo. Measurements of average tumor size for each group of animals were made on the tenth, fourteenth and eighteenth day following initial tumor implantation and dosing. Column I under each of the reported days gives the average tumor size in square millimeters. Column II under each of the reported days gives the number of surviving animals out of each test group. As can be seen from the data presented in Table 3, the average tumor size after fourteen days of treatment for the group receiving combination chemotherapy according to this invention was about one-half the average tumor size for the group receiving 5-FU alone. Moreover, after eighteen days of treatment, three of the five animals receiving combination chemotherapy were still alive, whereas all of the control animals and those receiving 5-FU alone had died.

Table 3

| Compound administered | Day 0 | | 10 | | 14 | | 18 | |
|---|---|---|---|---|---|---|---|---|
| | I | II | I | II | I | II | I | II |
| controls | — | 20/20 | 750 | 20/20 | 660 | 6/20 | — | 0/20 |
| 5 FU | — | 5/5 | 490 | 5/5 | 490 | 4/5 | — | 0/5 |
| 5 FU + A | — | 5/5 | 240 | 5/5 | 235 | 4/5 | 140 | 3/5 |

In a similar test, hamsters were infected with an aly fast tumor strain of bronchiogenic carcinoma of the lung. Cytoxan was administered to one group of infected animals at an intraperitoneal dose of 10 mg./kg. once daily. A compound of this invention, N-(2-phenyl-2-hydroxyethyl)-1,1-dimethyl-3-(4-hydroxyphenyl)propylaminium bromide (designated in Table 4 as A), caused a significant synergistic effect when co-administered with cytoxan. Cytoxan was administered i.p. once daily at 10 mg./kg. to the animals receiving the combination chemotherapy, and compound A was administered subcutaneously at the rate of 10 mg./kg. twice daily. The results of the test are presented in Table 4. It should be noted that the average tumor size for the animals receiving the combination of cytoxan and N-(2-phenyl-2-hydroxyethyl)-1,1-dimethyl-3-(4-hydroxyphenyl)propylaminium bromide was only 20 square millimeters after the nineteenth day of treatment, while the average tumor size for the group receiving cytoxan was about the same as that for the animals receiving placebo, namely about 1340 square millimeters.

Table 4

| Compound administered | Day 0 | | 8 | | 13 | | 19 | |
|---|---|---|---|---|---|---|---|---|
| | I | II | I | II | I | II | I | II |
| controls | — | 40/40 | 510 | 38/40 | 830 | 35/40 | 1360 | 9/40 |
| Cytoxan | — | 10/10 | 560 | 10/10 | 950 | 9/10 | 1340 | 6/10 |
| Cytoxan + A | — | 10/10 | 50 | 9/10 | 20 | 8/10 | 20 | 8/10 |

In a test designed to demonstrate the potentiating effect of the compounds of this invention on the oncolytic activity of the vinca alkaloids, mice having an implanted adenocarcinoma (CA 755 tumor) were administered placebo, vindesine, and the combination of vindesine and a compound of this invention, N-(2-phenyl-2-hydroxyethyl)-1,1-dimethyl-3-(4-aminocarbonylphenyl)propylaminium chloride (referred to in Table 4 as compound B). Vindesine was administered intraperitoneally at the rate of 0.6 mg./kg. on days 1, 5, 9 and 13 of the test. The animals receiving combination chemotherapy were administered vindesine intraperitoneally according to the above-indicated dosage regimen, and N-(2-phenyl-2-hydroxyethyl)-1,1-dimethyl-3-(4-aminocarbonylphenyl)propylaminium chloride was administered intraperitoneally at the rate of 7.5 mg./kg. twice daily for the first 15 days of the test. The results of the test are presented in Table 5. Under column I of each of the indicated days of measurement is given the average tumor size in square millimeters for each test group. Column II under each of the indicated days of measurement provides the number of tumor free animals out of those still living on that particular day in each test group. As can be seen from the data in Table 5, of the ten animals treated with the combination according to this invention, three were still living on day 40 of the test, and of those three, two were tumor free. In contrast, all of the control animals and those receiving vindesine alone had died by day 40, and none were tumor free after the thirteenth day of the test.

Table 5

| Compound administered | Day 0 | | 8 | | 13 | | 19 | | 26 | | 40 | |
|---|---|---|---|---|---|---|---|---|---|---|---|---|
| | I | II | I | II | I | II | I | II | I | II | I | II |
| controls | — | 0/30 | 190 | 0/30 | 410 | 0/30 | 620 | 0/23 | 640 | 0/3 | — | 0/0 |
| VDS | — | 0/10 | 35 | 3/9 | 170 | 0/8 | 300 | 0/8 | 620 | 0/6 | — | 0/0 |
| VDS + B | — | 0/10 | 20 | 6/8 | 15 | 6/7 | 25 | 5/7 | 180 | 3/7 | 220 | 2/3 |

5-Fluorouracil is essentially ineffective against the 755 adenocarcinoma in mice. However, when administered in combination with a compound of this invention, 5—FU becomes very effective. Such result is demonstrated by the data presented in Table 6. Mice were infected by an implantation of the 755 adenocarcinoma. Thirty animals were given placebo, ten were given 5-fluorouracil alone, and ten were given N-(2-phenyl-2-hydroxyethyl)-1,1-dimethyl-3-(4-aminocarbonylphenyl)propylaminium chloride in combination with 5-fluorouracil. Treatment in all cases was started five days after implantation of the tumor system. 5-Fluorouracil was administered intraperitoneally at the rate of 8 mg./kg. given once on days 5, 9 and 13 following implantation, both to the animals receiving 5—FU alone and the combination. The animals undergoing combination chemotherapy received, in addition to 5—FU, N-(2-phenyl-2-hydroxyethyl)-1,1-dimethyl-3-(4-aminocarbonylphenyl)propylaminium chloride (compound B) at the rate of 7.5 mg./kg. given subcutaneously twice daily on days 5 through 14 of the test. Table 6 presents the results of the test. Column I under each of the days of measurement reports the average tumor size in square millimeters of each test group. Column II under each of the days of measurement is the number of tumor free animals out of those still living on that day. The data show that combination chemotherapy utilizing a compound of this invention with 5-fluorouracil is effective in the treatment of the 755 adenocarcinoma, whereas 5-fluorouracil alone is ineffective.

Table 6

| Compound administered | Day 0 | | 5 | | 12 | | 17 | | 23 | | 29 | | 73 | |
|---|---|---|---|---|---|---|---|---|---|---|---|---|---|---|
| | I | II | I | II | I | II | I | II | I | II | I | II | I | II |
| controls | — | 0/30 | 100 | 0/30 | 400 | 0/30 | 750 | 0/22 | 700 | 0/6 | — | 0/0 | — | 0/0 |
| 5 FU | — | 0/10 | 95 | 0/10 | 350 | 0/10 | 630 | 0/9 | 680 | 0/6 | — | 0/0 | — | 0/0 |
| 5 FU + B | — | 0/10 | 70 | 0/10 | 100 | 0/10 | 80 | 1/10 | 120 | 1/9 | 500 | 1/7 | — | 0/0 |

Table 7 presents the results wherein total tumor remissions were observed. Mice were infected by implantation of a 755 adenocarcinoma. Thirty mice were held as controls and receive only placebo. Ten mice were administered 4 mg./kg. of 6-mercaptopurine (6 MP) intraperitoneally once per day on the fifth, ninth and thirteenth days following implantation. To another group of ten mice was administered N-(2-phenyl-2-hydroxyethyl)-1,1-dimethyl-3-(4-aminocarbonylphenyl)propylaminium chloride subcutaneously at the rate of 3.75 mg./kg. twice daily for ten days starting on the fifth day after implantation. Said administration was done in combination with 6 MP at the above-recited dose schedule. Table 7 reports the average tumor size of each group of test animals on the indicated days of measurement. Column II under each of the reported days is the number of animals which were tumor free out of those animals still living. It should be noted that by day 23, twenty-four of the thirty animals receiving placebo had died. All ten of the animals receiving 6-mercaptopurine alone had died by day 29. Of the ten animals receiving the combination of 6 MP and a compound of this invention, only four had died after 73 days following implantation, and all six animals which were living were tumor free.

Table 7

| Compound administered | Day 0 | | 5 | | 12 | | 17 | | 23 | | 29 | | 73 | |
|---|---|---|---|---|---|---|---|---|---|---|---|---|---|---|
| | I | II | I | II | I | II | I | II | I | II | I | II | I | II |
| controls | — | 0/30 | 100 | 0/30 | 400 | 0/30 | 730 | 0/30 | 680 | 0/6 | — | 0/0 | — | 0/0 |
| 6 MP | — | 0/10 | 130 | 0/10 | 370 | 0/10 | 570 | 0/10 | 650 | 0/8 | — | 0/0 | — | 0/0 |
| 6 MP + B | — | 0/10 | 65 | 0/10 | 50 | 0/10 | 25 | 0/10 | 20 | 6/8 | 15 | 6/8 | 0 | 6/6 |

A similar study was carried out with cytoxan at varying doses and the combination of cytoxan and N-(2-phenyl-2-hydroxyethyl)-1,1-dimethyl-3-(4-aminocarbonylphenyl)propylaminium chloride. A group of ten animals infected with the 755 adenocarcinoma were given cytoxan intraperitoneally at the rate of 25 mg./kg. once per day on days 5, 9 and 13 of the test. Another group of infected animals received cytoxan at 12.5 mg./kg. once per day on days 5, 9 and 13. A third group of infected animals received the combination of cytoxan, given i.p. at 12.5 mg./kg. on days 5, 9 and 13, and the compound of this invention (compound B) given subcutaneously twice daily on days 5 through 14 at the dose of 7.5 mg./kg. Table 8 presents the results of such study. As indicated by the data, all of the control animals and those receiving the oncolytic agent alone were dead after 73 days. In contrast, six of the ten animals which received combination chemotherapy were still living after 73 days of the test, and all of those still living were tumor free.

Table 8

| Compound administered | Day 0 | | 5 | | 12 | | 17 | | 23 | | 29 | | 73 | |
|---|---|---|---|---|---|---|---|---|---|---|---|---|---|---|
| | I | II | I | II | I | II | I | II | I | II | I | II | I | II |
| Controls | — | 0/30 | 80 | 0/30 | 390 | 0/30 | 730 | 0/22 | 700 | 0/6 | — | 0/0 | — | 0/0 |
| Cytoxan @ 25 mg./kg. | — | 0/10 | 80 | 0/10 | 60 | 1/10 | 60 | 4/10 | 200 | 2/9 | 570 | 0/4 | — | 0/0 |
| Cytoxan @ 12.5 mg./kg. | — | 0/10 | 130 | 0/10 | 190 | 0/10 | 300 | 0/10 | 600 | 1/9 | 800 | 0/4 | — | 0/0 |
| Cytoxan @ 12.5 mg./kg. + B | — | 0/10 | 70 | 0/10 | 40 | 2/10 | 25 | 7/9 | 10 | 7/7 | 30 | 6/7 | 0 | 6/6 |

As the foregoing test results indicate, the compounds provided by this invention are useful in potentiating the efficacy of antineoplastic agents. The compounds thus can be used in combination with known oncolytic drugs in the treatment of neoplastic diseases. A further aspect of this invention accordingly is a pharmaceutical formulation for treating mammals suffering from a neoplastic disease and in need of treatment.

The N-[2-phenyl (and 2-fluorophenyl)-2-hydroxyethyl]-1,1-dimethyl-3-(4-substituted phenyl)-propylamines provided herein can be formulated for convenient administration via the oral or parenteral routes. Formulations will generally contains from about 1 to about 50 percent by weight of a compound of this invention. The compounds can be admixed with any of a number of suitable pharmaceutical carriers, excipients and diluents. Typical carriers and diluents commonly used include dextrose, sucrose, starch powder, cellulose fiber, sorbitol, mannitol, polyvinylpyrrolidone, methyl cellulose, ethyl lactate, methyl hydroxybenzoate, silica, liquid paraffin and related excipients and carriers. The formulations can contain one or more of the compounds of this invention, and if desired, the formulation can contain an antineoplastic drug such as cytoxan, 6-mercaptopurine or the like so that the combination chemotherapy can be accomplished by the administration of an effective dose of the combination in a single form of the active drug and the potentiator. It is preferred, however, that the compounds of the invention be formulated separately from the antineoplastic drugs and that the two or more agents be administered separately.

For oral administration, a compound of this invention can be admixed with carriers such as starch and sucrose and molded into tablets or enclosed in gelatin capsules. Alternatively, the compounds can be formulated in the form of a syrup or suspension, for instance in sterile water, saline solution or the like. For parenteral administration, for instance via the intramuscular, subcutaneous or intravenous routes, the compounds can be admixed with carriers such as isotonic saline or glucose, or alternatively they can be dissolved in a suitable solvent such as water, placed in a vial and lyophilized to a powder. Such powder is ready for re-constitution by the addition of sterile water or saline for ready intramuscular administration. The compounds can also be formulated and placed in the form of suppositories, buccal seals, and the like, for convenient time release administration.

According to the method of treatment, a compound having the above general formula is administered to a subject suffering from a neoplastic disease and in need of treatment in combination with an antineoplastic agent known to be useful in the treatment of such disease. The dosage contemplated for the compounds of this invention is an amount sufficient to potentiate the known oncolytic agent. Such dosage may vary depending upon the particular neoplasm undergoing treatment, the antineoplastic agent being potentiated, the severity of the affliction, the route of administration and related factors. A compound of this invention will, however, routinely be administered at a dosage from about 0.5 to about 50 mg./kg. of animal body weight. A preferred dosage will be from about 1 to about 20 mg./kg. administered from 1 to about 4 times per day. As hereinbefore noted, it is preferred to formulate and administer the potentiating agents of this invention separately from the antineoplastic agent or agents. For example, a compound such as N-(2-phenyl-2-hydroxyethyl)-1,1-dimethyl-3-(4-di-methylaminocarbonylphenyl)propylaminium acetate can be formulated for oral administration at a dose of about 10 mg./kg. administered about twice each day. An oncolytic agent such as cytoxan or the like then can be administered parenterally in the normal fashion, either at the normal clinical dosage rate or at a suboptimal level. For example, an agent such as cytoxan generally is administered at an oral dose of about 1 to 5 mg./kg. once each day for the treatment of solid tumors such as neuroblastoma, adenocarcinoma of the ovary, retinoblastoma and the like. Adverse reactions commonly encountered with continued cytoxan therapy include secondary malignancy development, leukopenia, nausea, anorexia, oral mucosal ulceration and jaundice. According to this invention, a compound having the above formula is administered in combination with cytoxan at a dose of from 1 to 5 mg./kg., or at a suboptimal dose, with the net result that the overall effect of the known oncolutic agent on malignant tissue is enhanced.

In an effort to more fully describe specific aspects of the invention, the following detailed Examples are provided. The Examples are by no means intended to limit the invention and should not be so construed.

Preparation 1

1,1-Dimethyl-3-(4-methoxyphenyl)propylamine

A cold solution of 56 g. of sodium cyanide in 125 ml. of acetic acid was stirred and diluted by the addition of a solution of 140 ml. of conc. sulfuric acid containing 125 ml. of acetic acid. To the stirred acid solution was added 170.8 g. of 1,1-dimethyl-1-hydroxy-3-(4-methoxyphenyl)propane portionwise over ten minutes. Following complete addition of the alcohol, the reaction mixture was heated to 75°C. for thirty minutes and then cooled to 25°C. and stirred for an additional two hours. The reaction mixture next was poured into 400 ml. of ice water and made neutral by the addition of sodium carbonate. The aqueous solution was extracted several times with diethyl ether, and the ethereal extracts were combined, washed with water and dried. Removal of the solvent by evaporation under reduced pressure provided 142.8 g. of N-formyl-1,1-dimethyl-3-(4-methoxyphenyl)propylamine.

The latter compound was dissolved in 1000 ml. of 2.7 N hydrochloric acid and the acid reaction

mixture was heated at 100°C. for twelve hours. After cooling the acidic reaction mixture, it was added to 500 ml. of water, and then washed with ethyl acetate. The aqueous layer was made alkaline by the addition of sodium hydroxide, and the alkaline reaction mixture was extracted with diethyl ether. The ethereal extracts were combined, washed with water and dried. Evaporation of the solvent and distillation of the product afforded 72.9 g. of 1,1-dimethyl-3-(4-methoxyphenyl)propylamine. B.P. 110—120°C. at 133.3 N/$m^2$ (1.0 torr).

Preparation 2

Following the procedure set forth in Preparation 1, 1,1-dimethyl-1-hydroxy-3-(4-hydroxycarbonylphenyl)propane was reacted with sodium cyanide to provide, after hydrolysis of the intermediate N-formyl amine, 1,1-dimethyl-3-(4-hydroxycarbonylphenyl)propylamine. The amine thus formed was converted to the hydrochloride salt and the salt was reacted with thionyl chloride to provide 1,1-dimethyl-3-(4-chlorocarbonylphenyl)propylamine. The acid chloride so produced was reacted with an excess of ammonia to provide 1,1-dimethyl-3-(4-aminocarbonylphenyl)propylamine. In a similar fashion, the acid chloride was reacted with slight excesses of methylamine and dimethylamine to provide, respectively, 1,1-dimethyl-3-(4-methylaminocarbonylphenyl)propylamine and 1,1-dimethyl-3-(4-dimethylaminocarbonylphenyl)propylamine.

Example 1

N-(2-Phenyl-2-hydroxyethyl)-1,1-dimethyl-3-(4-hydroxyphenyl)propylamine

To a stirred solution of 72.9 g. of 1,1-dimethyl-3-(4-methoxyphenyl)propylamine in 1000 ml. of diethyl ether was added dropwise over one hour a solution of 37.8 g. of 2-bromoacetophenone in 450 ml. of diethyl ether. When the addition was complete, the reaction mixture was heated to reflux and stirred for one week. The reaction mixture was next cooled to room temperature and filtered to provide 53 g. of unreacted amine as the hydrobromide salt. The filtrate was washed with water, dried, and the solvent was removed by evaporation to afford, after crystallization from ethanol and ethyl acetate, 20 g. of N-(2-phenyl-2-oxoethyl)-1,1-dimethyl-3-(4-methoxyphenyl)propylamine. M.P. 180.7—181.5°C.

The amine thus formed was dissolved in 200 ml. of aqueous hydrobromic acid. The acidic reaction mixture was heated to reflux and stirred for twelve hours. The mixture then was cooled to room temperature, whereupon the crude product of the reaction crystallized. The crystalline material was collected by filtration and recrystallized twice from ethanol to afford 13.0 g. of N-(2-phenyl-2-oxoethyl)-1,1-dimethyl-3-(4-hydroxyphenyl)propylaminium bromide. M.P. 213°C. (dec.).

A solution of 13.0 g. of N-(2-phenyl-2-oxoethyl)-1,1-dimethyl-3-(4-hydroxyphenyl)-propylaminium bromide in 235 ml. of a ninety percent solution of ethanol in water containing 2 g. of five percent palladium on carbon was stirred for two hours and fifteen minutes under a hydrogen atmosphere of 60 psi. The reaction mixture then was filtered and the filtrate was concentrated by evaporation of the solvent to provide a solid residue. The solid thus formed was crystallized from ethyl acetate to afford 5.5 g. of N-(2-phenyl-2-hydroxyethyl)-1,1-dimethyl-3-(4-hydroxyphenyl)-propylaminium bromide. M.P. 169—170°C.

Analysis calc. for $C_{19}H_{26}NO_2Br$
Theory: C, 60.00; H, 6.89; N, 3.68.
Found: C, 60.17; H, 6.59; N, 3.61.

The amine hydrobromide was next dissolved in 50 ml. of methanol and stirred at room temperature while a solution of excess hydrogen chloride in diethyl ether was added in one portion. The amine hydrochloride salt precipitated out of solution and was collected by filtration. The solid product was recrystallized three times from methanol and ethyl acetate to provide N-(2-phenyl-2-hydroxyethyl)-1,1-dimethyl-3-(4-hydroxyphenyl)propylaminium chloride. M.P. 180—182°C.

Analysis calc. for $C_{19}H_{26}NO_2Cl$
Theory: C, 67.91; H, 7.80; N, 4.17.
Found: C, 68.13; H, 7.59; N, 4.22.

Example 2

N-[2-(2-fluorophenyl)-2-hydroxyethyl]-1,1-dimethyl-3-(4-hydroxyphenyl)propylamine

To a stirred refluxing solution of 10.0 g. of 1,1-dimethyl-3-(4-hydroxyphenyl)propylamine in 100 ml. of ethanol was added dropwise over thirty minutes 9.1 g. of *o*-fluorostyrene oxide. The reaction mixture was heated at reflux for twelve hours following complete addition. After cooling the reaction mixture to room temperature, the solvent was removed therefrom by evaporation under reduced pressure to provide the product as an oil. The oil was dissolved in diethyl ether and extracted three times with ten percent aqueous hydrochloric acid and then twice with water. The aqueous acid extracts were combined, made alkaline by the addition of sodium hydroxide, and then extracted with fresh diethyl ether. The ethereal extracts were combined, washed with water and dried. Concentration of the solution by evaporation of a portion of the solvent effected crystallization of the product as a white

solid. The solid was collected by filtration and recrystallized three times from ethyl acetate and once from methanol. The crystalline product next was dissolved in fresh ethyl acetate and added to an ethereal solution of hydrogen chloride, whereupon a crystalline solid precipitated and was collected by filtration and dried to provide 3.4 g. of N-[2-(2-fluorophenyl)-2-hydroxyethyl]-1,1-dimethyl-3-(4-hydroxyphenyl)propylaminium chloride. M.P. 157—159.5°C.

Analysis calc. for $C_{19}H_{24}NO_2FCl$
Theory: C, 64.49; H, 7.12; N, 3.96.
Found: C, 64.57; H, 7.19; N, 4.20.

Example 3

N-(2-Phenyl-2-hydroxyethyl)-1,1-dimethyl-3-(4-aminocarbonylphenyl)propylamine

A solution of 3.9 g. of 1,1-dimethyl-3-(4-aminocarbonylphenyl)propylamine in 150 ml. of ethanol containing 2.8 g. of styrene oxide was heated to reflux and stirred for twelve hours. After cooling the reaction mixture to room temperature, the solvent was removed therefrom by evaporation to provide a solid residue. The solid was crystallized from 100 ml. of hot diethyl ether to provide 3.3 g. of crystalline product. The product thus formed was recrystallized twice from hot acetone to afford 1.98 g. of N-(2-phenyl-2-hydroxyethyl)-1,1-dimethyl-3-(4-aminocarbonylphenyl)propylamine. M.P. 146—150°C.

Analysis calc. for $C_{20}H_{26}N_2O_2$
Theory: C, 73.59; H, 8.03; N, 8.58.
Found: C, 73.40; H, 7.91; N, 8.33.

The amine base thus formed was dissolved in methanol and added to a solution of hydrogen chloride in diethyl ether. The solid precipitate which formed was collected and recrystallized from 30 ml. of acetone and 5 ml. of ethanol to provide 1.79 g. of N-(2-phenyl-2-hydroxyethyl)-1,1-dimethyl-3-(4-aminocarbonylphenyl)propylaminium chloride. M.P. 204—206°C.

Analysis calc. for $C_{20}H_{27}N_2O_2Cl$
Theory: C, 66.19; H, 7.50; N, 7.72; Cl, 9.77.
Found: C, 66.05; H, 7.42; N, 7.53; Cl, 10.06.

Example 4

Following the procedure set forth in Example 3, 4.6 g. of *o*-fluorostyrene oxide was reacted with 5.8 g. of 1,1-dimethyl-3-(4-aminocarbonylphenyl)propylamine to provide, after crystallization from methanol and ethyl acetate, 2.0 g. of N-[2-(2-fluorophenyl)-2-hydroxyethyl]-1,1-dimethyl-3-(4-aminocarbonylphenyl)propylamine.

Analysis calc. for $C_{20}H_{25}N_2O_2F$
Theory: C, 69.74; H, 7.32; N, 8.13.
Found: C, 69.58; H, 7.12; N, 8.28.

The amine base thus formed was converted to its hydrochloride salt by reaction with hydrogen chloride in diethyl ether to give 1.2 g. of N-[2-(2-fluorophenyl)-2-hydroxyethyl]-1,1-dimethyl-3-(4-aminocarbonylphenyl)propylaminium chloride. M.P. 227—230°C.

Analysis calc. for $C_{20}H_{26}N_2O_2ClF$
Theory: C, 63.07; H, 6.88; N, 7.35.
Found: C, 63.21; H, 7.12; N, 7.45.

Examples 5—6

By following the procedure set out in Example 3, styrene oxide was reacted with the appropriate 1,1-dimethyl-3-(4-substituted phenyl)propylamine to provide, after salt formation, the following compounds:

N - (2 - phenyl - 2 - hydroxyethyl) - 1,1 - dimethyl - 3 - (4 - methylaminocarbonylphenyl)-propylaminium chloride. M.P. 219—220°C.

N - (2 - phenyl - 2 - hydroxyethyl) - 1,1 - dimethyl - 3 - (4 - dimethylaminocarbonyl-phenyl)propylaminium oxalate, M.P. 237—240°C.

Example 7

R-N-(2-Phenyl-2-hydroxyethyl)-1,1-dimethyl-3-(4-hydroxyphenyl)propylamine

A solution of 9.19 g. of 1,1-dimethyl-3-(4-hydroxyphenyl)propylamine in 40 ml. of dimethylformamide containing 7.80 g. of R-mandelic acid and 6.92 g. of 1-hydroxybenzotriazole was stirred and cooled to —30°C. in an ice/acetone bath. To the cold reaction mixture was added dropwise over thirty

minutes a solution of 10.57 g. of N,N'-dicyclohexylcarbodiimide in 35 ml. of dimethylformamide. Following complete addition, the reaction mixture was allowed to stand at −5°C. for twelve hours, after which time it was filtered. The filtrate was concentrated to dryness by evaporation of the solvent under reduced pressure, and the residue thus formed was dissolved in 200 ml. of ethyl acetate and washed with aqueous sodium carbonate, with 3 N hydrochloric acid, and finally with saturated aqueous sodium chloride solution. The organic layer next was separated and dried and the solvent was removed by evaporation to provide 9.0 g. of R-(2-phenyl-2-hydroxy-1-oxoethyl)-1,1-dimethyl-3-(4-hydroxyphenyl)propylamine as a foam. The foam was purified by crystallization from ethyl acetate. M.P. 159—170°C.

Analysis calc. for $C_{19}H_{23}NO_2$
Theory: C, 72.82; H, 7.40; N, 4.47.
Found: C, 72.57; H, 7.34; N, 4.54.

The optically active amide (2.02 g.) thus formed was dissolved in 80 ml. of benzene containing 2.12 ml. of triethylamine and 1.33 g. of trimethylchlorosilane. The precipitate triethylamine hydrochloride was removed by filtration, and the filtrate was then concentrated to dryness by evaporation of the solvent. The residue was dissolved in 40 ml. of tetrahydrofuran and stirred while 16 ml. of 1N diborane in tetrahydrofuran was added to the reaction mixture in one portion. The resulting reaction mixture was stirred at room temperature for twelve hours, and then was diluted by the addition of 20 ml. of methanol. With continued stirring, 50 ml. of a solution of hydrogen chloride in diethyl ether was added to the reaction mixture, and the solution then was heated to 60°C. for twenty minutes. Removal of the solvent by evaporation then afforded a solid residue, which upon crystallization from 25 ml. of hot ethyl acetate and methanol provided 840 mg. of R-N-(2-phenyl-2-hydroxyethyl)-1,1-dimethyl-3-(4-hydroxyphenyl)propylaminium chloride. M.P. 184—192°C.

Analysis calc. for $C_{19}H_{26}NO_2Cl$
Theory: C, 67.94; H, 7.80; N, 4.17; Cl, 10.56.
Found: C, 68.14; H, 7.65; N, 3.93; Cl, 10.72.

Optical Rotation 7 mg./ml. $CH_3OH$: $[\alpha]_D = -26.6°$; $[\alpha]_{365} = -87.3°$.

Example 8

By following the general procedure set out in Example 3, 1,1-dimethyl-3-(4-aminocarbonylphenyl)propylamine was reacted with optically active R-*o*-fluorostyrene oxide to provide R-N-[2-(2-fluorophenyl)-2-hydroxyethyl]-1,1-dimethyl-3-(4-aminocarbonylphenyl)propylamine.

Example 9

N-(2-Phenyl-2-hydroxyethyl)-1,1-dimethyl-3-(4-methanesulfonylaminophenyl)propylamine, hydrochloride

(a) *N-[4-(3-Hydroxy-3-methyl-1-butenyl)phenyl]methanesulfonamide*

A mixture of 0.80 g. of palladium chloride and 2.5 g. of triphenylphosphine in 150 ml. of dimethylformamide under an atmosphere of nitrogen was heated with stirring at 100°C. until all of the palladium chloride had reacted. The mixture was cooled to 80°C. and 30 g. of sodium bicarbonate, 71.3 g. of *p*-bromophenylmethylsulfonamide, 35 g. of 2-methyl-3-butene-2-ol and 0.5 ml. of triethylamine was added. Stirring was continued and the reaction temperature raised to 120°C. After three and a half hours, the mixture was cooled, poured over ice and extracted repeatedly with ethyl acetate. The combined organic extracts were washed with water, dried over anhydrous sodium sulfate and filtered. The solvent was recovered under reduced pressure. The residue was induced to solidify by repeated treatment with a mixture of benzene, ether and hexane. The yield was 12.6 g. of a pale yellow solid.

Analysis calc. for $C_{12}H_{17}NO_3S$
Theory: C, 56.45; H, 6.71; N, 5.49.
Found: C, 56.58; H, 6.63; N, 5.23.

(b) *N-[4-(3-Hydroxy-3-methylbutyl)phenyl]methanesulfonamide*

A solution of 12.5 g. of the butenylsulfonamide from (a) in 135 ml. of ethyl acetate was hydrogenated at 60 p.s.i. in the presence of 3 g. of Raney nickel. The catalyst was recovered by filtration, and the solvent was removed under reduced pressure. The oily residue spontaneously crystallized. The product was purified by recrystallization from a mixture of ethyl acetate and hexane to give 8.7 g. of solid, m.p. 100—106°C.

Analysis calc. for $C_{12}H_{19}NO_3S$
Theory: C, 56.01; H, 7.44; N, 5.44.
Found: C, 55.92; H, 7.19; N, 5.18.

(c) *N-[4-(3-Formamido-3-methylbutyl)phenyl]methanesulfonamide*

To an ice-cooled and stirred mixture of 3.0 g. of sodium cyanide and 20 ml. of glacial acetic acid was added dropwise a solution of 20 ml. of concentrated sulfuric acid and 20 ml. of glacial acetic acid, followed by 12.2 g. of the methanesulfonamide alcohol from (b) in small portions. The reaction mixture was heated at 60°C. for two hours and then stirred at 25°C. for sixteen hours. This slurry was poured onto ice and extracted several times with ethyl acetate. The combined organic layers were washed consecutively with water, an aqueous solution of sodium bicarbonate, and water. The solvent was removed under reduced pressure. The oily residue was crystallized from ethyl acetate to give 7.7 g. of solid, m.p. 117—119°C.

Analysis calc. for $C_{13}H_{20}N_2O_3S$
Theory: C, 54.91; H, 7.09; N, 9.85.
Found: C, 54.84; H, 7.10; N, 9.71.

(d) *N-[4-(3-Amino-3-methylbutyl)phenyl]methanesulfonamide hydrochloride*

A mixture of 7.54 g. of the formamide from (c) 12 ml. of ethanol and 50 ml. of 3N hydrochloric acid was heated to reflux for three and a half hours. The solvents were removed under reduced pressure to give 7.51 g. of product, m.p. 218—219°C.

Analysis calc. for $C_{12}H_{21}ClN_2O_2S$
Theory: C, 49.22; H, 7.23; N, 9.57.
Found: C, 49.43; H, 7.36; N, 9.30.

The hydrochloride salt was converted to 6.17 g. of the free base by treatment with aqueous sodium carbonate. The amine was dissolved in 200 ml. of 1-butanol and 4.4 g. of styrene oxide and refluxed for ten hours. The solvent was removed under reduced pressure to give 10.4 g. of solid residue. Recrystallization from acetonitrile gave 5.24 g. of solid, m.p. 153—156°C.

The amino alcohol was converted to the hydrochloride salt. Recrystallization from a mixture of methanol and ether gave 4.41 g. of title product, m.p. 208—210°C.

Analysis calc. for $C_{20}H_{29}ClN_2O_3S$
Theory: C, 58.17; H, 7.08; N, 6.78; Cl, 8.58.
Found: C, 58.32; H, 7.23; N, 6.74; Cl, 8.65.

Example 10

N-(2-Phenyl-2-hydroxyethyl)-1,1-dimethyl-3-(4-acetylaminophenyl)propylamine, hydrochloride

(a) *N-[4-(3-Hydroxy-3-methyl-1-butenyl)phenyl]acetamide*

A mixture of 1.60 g. of palladium chloride and 5.0 g. of triphenylphosphine in 350 ml. of dimethylformamide under an atmosphere of nitrogen was heated with stirring until all of the palladium chloride had reacted. The mixture was cooled to 80°C. and 42.8 g. of *p*-bromoacetanilide, 20 g. of sodium bicarbonate, 25.8 g. of 3-hydroxy-3-methylbutene, 1 ml. of triethylamine and 100 ml. of dimethylformamide were added. Stirring was continued whilst the reaction temperature was raised to 120°C. After seven hours the mixture was poured onto ice and extracted with ethyl acetate. The organic extracts were washed with water and the solvent was removed under reduced pressure. The solid residue was filtered and washed with a mixture of ether and hexane to give 17 g. of tan solid, m.p. 140—142°C.

(b) *N-[4-(3-Hydroxy-3-methylbutyl)phenyl]acetamide*

A solution of 15.0 g. of the olefin from (a) in 100 ml. of ethanol and 100 ml. of ethyl acetate was hydrogenated in the presence of Raney nickel. The catalyst was removed by filtration and the solvents were removed under reduced pressure to give 14.8 g. of solid material. Recrystallization from a mixture of ethyl acetate and acetone gave 12.0 g. of N-[4-(3-hydroxy-3-methylbutyl)phenyl]acetamide, m.p. 128—130°C.

(c) *N-[4-(3-Formamido-3-methylbutyl)phenyl]acetamide*

A mixture of 12.3 g. of sodium cyanide and 35 ml. of glacial acetic acid was stirred and cooled in an ice bath. After fifteen minutes a solution of 40 ml. of concentrated sulfuric acid and 35 ml. of glacial acetic acid was added dropwise. Upon completion of the addition 50 g. of the alcohol from the preceding example was added in small portions. The mixture was stirred at ambient temperature for

sixty hours, heated at 55°C. for one-half hour and poured onto ice. The reaction was made basic by the addition of sodium carbonate and extracted with ethyl acetate. Evaporation of the solvent after washing with water afforded a solid which was purified by recrystallization from a mixture of methanol and ethyl acetate. There was obtained 43.7 g. of N-[4-(3-formamido-3-methylbutyl)phenyl]acetamide, m.p. 152.5—155°C.

Analysis calc. for $C_{14}H_{20}N_2O_2$
Theory: C, 67.72; H, 8.12; N, 11.28.
Found: C, 67.58; H, 8.03; N, 11.09.

(d) *4-(4-Aminophenyl)-2-amino-2-methylbutane, dihydrochloride*

A mixture of 43.6 g. of the formamide from (c), 300 ml. of ethanol and 1.2 l. of 3N hydrochloric acid was refluxed for four hours. The reaction mixture was evaporated to dryness under reduced pressure. The crude solid was treated with ether, filtered and dried to give 47.3 g. of dihydrochloride salt, m.p. 270°C. (dec.).

Analysis calc. for $C_{11}H_{20}Cl_2N_2$
Theory: C, 52.60; H, 8.03; N, 11.15.
Found: C, 52.36; H, 7.82; N, 11.15.

(e) *N-[4-(3-Amino-3-methylbutyl)phenyl]acetamide hydrochloride*

The pH of a solution of 46.2 g. of the dihydrochloride salt from (d) in 600 ml. of water was adjusted to 4.2 by the addition of a one percent solution of sodium carbonate. A solution of 22.1 g. of acetic anhydride in 100 ml. of tetrahydrofuran was added dropwise whilst the pH of the solution was kept constant at 4.2 by continued slow addition of a one percent solution of sodium carbonate. The reaction mixture was stirred at 25°C. for sixteen hours. The tetrahydrofuran was removed under reduced pressure, and the solid was removed by filtration and dried to give 48.8 g. of the monohydrate hydrochloride salt, m.p. 236—239°C.

Analysis calc. for $C_{13}H_{21}ClN_2O \cdot H_2O$
Theory: C, 56.83; H, 8.44; N, 10.19.
Found: C, 55.73; H, 7.45; N, 9.73.

Titration: calc. molecular weight: 274.5.
found: 272.

(f) *N-[4-[3-[(2-Hydroxy-2-phenylethyl)amino]-3-methylbutyl]phenyl]acetamide*

By treatment of 8.0 g. of the monohydrate hydrochloride salt of (e) with sodium carbonate there was obtained 6.2 g. of the free amine. This amine was dissolved in 1-butanol and heated to reflux. To this solution was added 3.7 g. of styrene oxide and reflux was continued for eight hours. The solvent was removed under reduced pressure, and the solid residue was treated with ether and filtered to give 5.4 g. of crystalline material, m.p. 142—145°C. Recrystallization from ethyl acetate afforded 4.8 g. of the amino alcohol, m.p. 146—148°C.

Analysis calc. for $C_{21}H_{28}N_2O_2$
Theory: C, 74.08; H, 8.29; N, 8.23.
Found: C, 73.88; H, 8.04; N, 7.98.

The solid free amine from the above reaction was dissolved in methanol and treated with an excess of an ethereal solution of hydrogen chloride. The solvent was removed under reduced pressure, and the solid residue was recrystallized from a mixture of ethanol and ethyl acetate to afford 1.4 g. of title product, m.p. 204—209°C.

Analysis calc. for $C_{21}H_{29}ClN_2O_2$
Theory: C, 66.92; H, 7.76; N, 7.43.
Found: C, 66.73; H, 7.56; N, 7.44.

Example 11

The following formulation was prepared for use in the treatment of solid tumors.

| | |
|---|---|
| N-(2-phenyl-2-hydroxyethyl)-1,1-dimethyl-3-(4-aminocarbonylphenyl)propylaminium chloride | 100 mg. |
| starch | 300 mg. |
| lactose | 100 mg. |
| | 500 mg. |

The above ingredients are thoroughly mixed and the mixture is lubricated with 1% magnesium stearate and compressed into tablets. Such tablets are administered at the rate of about 1 to 2 tablets per day for a patient weighing about 60 kg. in combination with an effective dose of cytoxan at about 2 mg./kg. administered orally once daily for the therapy of solid neoplasms such as neuroblastoma and adenocarcinoma of the ovary.

Example 12

Preparation for Parenteral Solution

| | |
|---|---|
| N-(2-phenyl-2-hydroxyethyl)-1,1-dimethyl-3-(4-hydroxyphenyl)-propylaminium chloride | 100 mg. |
| Benzyl alcohol | 0.9% w/v |
| Dextrose Injection, U.S.P. (5% dextrose) | 4 ml. |
| Isotonic saline (0.9% sodium chloride) | 2 ml. |

The above solution is administered to a subject weighing about 60 kg. at the rate of from 1 to 3 times per day in combination with an agent such as 5-fluorouracil at a suboptimal dose and continues until the cell count of the cerebrospiral fluid returns to normal. The formulation is well suited to the treatment of solid tumors such as osteogenic sarcoma and rhabdomyosarcoma.

Example 13

Formulation of intravenous administration

| | |
|---|---|
| R-N-[2-(2-fluorophenyl)-2-hydroxyethyl]-1,1-dimethyl-3-(4-aminocarbonylphenyl)-propylamine | 250 mg. |
| Isotonic saline | 50 ml. |

The above solution is administered once or twice a day in combination with a normal dosage regimen of an agent such as 6-mercaptopurine to a subject weighing from about 50 to about 70 kg. Neoplasms to be treated with the formulation include solid tumors of the breast, colon, stomach, pancreas, ovary, urinary bladder and the like.

**Claims**

1. A compound of the formula (I):

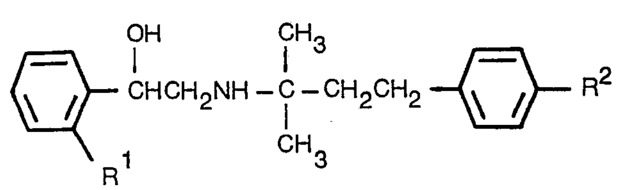

(I)

wherein:

$R^1$ is hydrogen or fluorine;

$R^2$ is hydroxy, aminocarbonyl, methylaminocarbonyl, dimethylaminocarbonyl, acetylamino or methanesulfonylamino;

or a pharmaceutically-acceptable acid addition salt thereof.

2. N-(2-Phenyl-2-hydroxyethyl)-1,1-dimethyl-3-(4-hydroxyphenyl)propylamine.
3. N-(2-Phenyl-2-hydroxyethyl)-1,1-dimethyl-3-(4-aminocarbonylphenyl)propylamine.
4. N-(2-Phenyl-2-hydroxyethyl)-1,1-dimethyl-3-(4-methylaminocarbonylphenyl)propylamine.
5. N-(2-Phenyl-2-hydroxyethyl)-1,1-dimethyl-3-(4-dimethylaminocarbonylphenyl)propylamine.
6. N-(2-Phenyl-2-hydroxyethyl)-1,1-dimethyl-3-(4-acetylaminophenyl)propylamine.
7. N-(2-Phenyl-2-hydroxyethyl)-1,1-dimethyl-3-(4-methanesulfonylaminophenyl)propylamine.
8. A compound as claimed in any one of Claims 1 to 7 which is the R-optical isomer.
9. A pharmaceutical formulation comprising a compound of formula (I) as claimed in any one of claims 1 to 8, or a pharmaceutically-acceptable acid addition salt thereof, associated with a pharmaceutically-acceptable carrier therefor.
10. A pharmaceutical formulation as claimed in claim 9 which also comprises an oncolytic agent.
11. A pharmaceutical formulation as claimed in claim 10, wherein the oncolytic agent is selected from cytoxan, 5-fluorouracil, vindesine, vincristine, vinblastine, methotrexate and 6-mercaptopurine.
12. A compound of formula (I) as claimed in any one of Claims 1 to 8, or a pharmaceutically-acceptable acid addition salt thereof, for use in the potentiation of the oncolytic effect of an antineoplastic agent.
13. A process for preparing a compound of formula (I) as claimed in any one of Claims 1 to 8 which comprises

(a) reducing a carbonyl compound of formula (II)

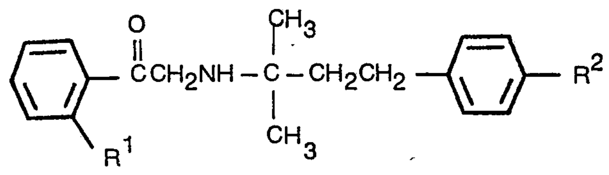

(II)

where $R^1$ and $R^2$ are as defined in Claim 1; or

(b) reacting a styrene oxide derivative of formula:

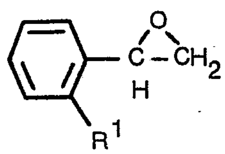

with an amine of formula:

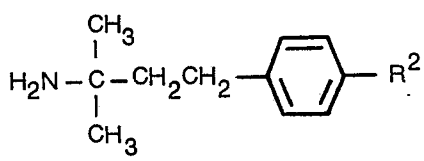

where $R^1$ and $R^2$ are as defined in Claim 1, and

(c) if appropriate, resolving a racemate formed by reaction (a) or (b) into the R-isomer.

**Revendications**

1. Composé répondant à la formule (I):

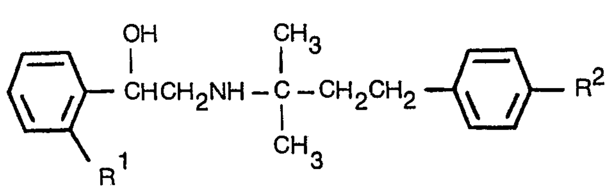

(I)

dans laquelle

$R^1$ représente un atome d'hydrogène ou un atome de fluor;

$R^2$ représente un groupe hydroxy, un groupe aminocarbonyle, un groupe méthylaminocarbonyle, un groupe diméthylaminocarbonyle, un groupe acétylamino ou un groupe méthane-sulfonylamino; ou un sel d'addition d'acide pharmaceutiquement acceptable de ce composé.

2. La N-(2-phényl-2-hydroxyéthyl)-1,1-diméthyl-3-(4-hydroxyphényl)propylamine.

3. La N-(2-phényl-2-hydroxyéthyl)-1,1-diméthyl-3-(4-aminocarbonylphényl)propylamine.

4. La N-(2-phényl-2-hydroxyéthyl)-1,1-diméthyl-3-(4-méthylaminocarbonylphényl)propylamine.

5. La N-(2-phényl-2-hydroxyéthyl)-1,1-diméthyl-3-(4-diméthylaminocarbonylphényl)propylamine.

6. La N-(2-phényl-2-hydroxyéthyl)-1,1-diméthyl-3-(4-acétylaminophényl)propylamine.

7. La N-(2-phényl-2-hydroxyéthyl)-1,1-diméthyl-3-(4-méthane-sulfonylaminophényl)propylamine.

8. Composé suivant l'une quelconque des revendications 1 à 7, ce composé étant l'isomère optique R.

9. Formulation pharmaceutique comprenant un composé de formule (I) suivant l'une quelconque des revendications 1 à 8 ou un sel d'addition d'acide pharmaceutiquement acceptable de ce composé en association avec un support pharmaceutiquement acceptable pour ce composé ou ce sel.

10. Formulation pharmaceutique suivant la revendication 9, caractérisée en ce qu'elle comprend également un agent oncolytique.

11. Formulation pharmaceutique suivant la revendication 10, caractérisée en ce que l'agent oncolytique est choisi parmi le cytoxane, le 5-fluorouracile, la vindésine, la vincristine, la vinblastine, le méthotrexate et la 6-mercaptopurine.

12. Composé répondant à la formule (I) suivant l'une quelconque des revendications 1 à 8 ou un sel d'addition d'acide pharmaceutiquement acceptable de ce composé en vue de l'utiliser pour la potentialisation de l'effet oncolytique d'un agent antinéoplastique.

13. Procédé de préparation d'un composé de formule (I) suivant l'une quelconque des revendications 1 à 8, caractérisé en ce qu'il comprend les étapes qui consistent à:

(a) réduire un composé carbonyle de formule (II):

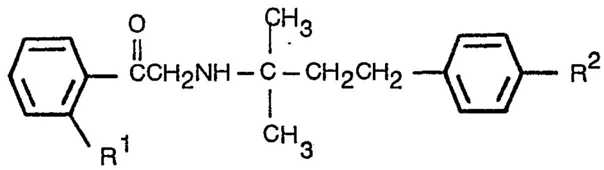 (II)

dans laquelle $R^1$ et $R^2$ ont les significations définies dans la revendication 1; ou

(b) faire réagir un dérivé d'oxyde de styrène de formule:

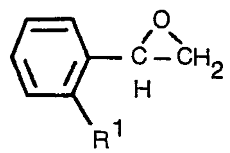

avec une amine de formule:

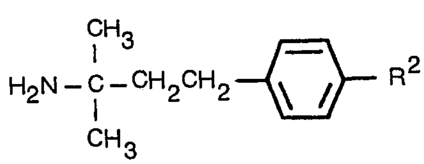

où $R^1$ et $R^2$ ont les significations définies dans la revendication 1, et

(c) si cela est indiqué, déboubler un racémate formé par la réaction (a) ou (b) en isomère R.

**Patentansprüche**

1. Phenethanolamine der allgemeinen Formel (I)

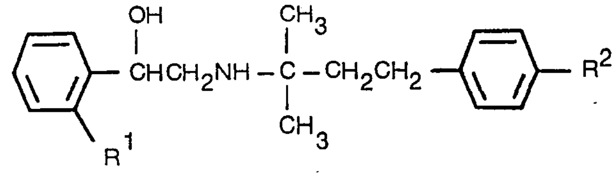 (I)

worin

$R^1$ Wasserstoff oder Fluor ist und

$R^2$ Hydroxy, Aminocarbonyl, Methylaminocarbonyl, Dimethylaminocarbonyl, Acetylamino und Methansulfonylamino bedeutet,

und die pharmazeutisch unbedenklichen Säureadditionssalze hiervon.

2. N-(2-Phenyl-2-hydroxyethyl)-1,1-dimethyl-3-(4-hydroxyphenyl)propylamin.

3. N-(2-Phenyl-2-hydroxyethyl)-1,1-dimethyl-3-(4-aminocarbonylphenyl)propylamin.

4. N-(2-Phenyl-2-hydroxyethyl)-1,1-dimethyl-3-(4-methylaminocarbonylphenyl)propylamin.

5. N-(2-Phenyl-2-hydroxyethyl)-1,1-dimethyl-3-(4-dimethylaminocarbonylphenyl)propylamin.

6. N-(2-Phenyl-2-hydroxyethyl)-1,1-dimethyl-3-(4-acetylaminophenyl)propylamin.

7. N-(2-Phenyl-2-hydroxyethyl)-1,1-dimethyl-3-(4-methansulfonylaminophenyl)propylamin.

8. Verbindung nach einem der Ansprüche 1 bis 7, dadurch gekennzeichnet, daß es sich hierbei um das optische Isomer R handelt.

9. Pharmazeutische Formulierung, dadurch gekennzeichnet, daß sie eine Verbindung der Formel (I) nach einem der Ansprüche 1 bis 8 oder ein pharmazeutisch unbedenkliches Säureadditionssalz hiervon in Verbindung mit einem pharmazeutisch unbedenklichen Träger hierfür enthält.

10. Pharmazeutische Formulierung nach Anspruch 9, dadurch gekennzeichnet, daß sie ferner auch ein oncolytisches Mittel enthält.

11. Pharmazeutische Formulierung nach Anspruch 10, dadurch gekennzeichnet, daß sie als oncolytisches Mittel Cytoxan, 5-Fluoruracil, Vindesin, Vincristin, Vinblastin, Methotrexat und/oder 6-Mercaptopurin enthält.

12. Verwendung einer Verbindung der Formel (I) nach einem der Ansprüche 1 bis 8 oder eines pharmazeutisch unbedenklichen Säureadditionssalzes hiervon zur Potenzierung der oncolytischen Wirksamkeit eines antineoplastischen Mittels.

13. Verfahren zur Herstellung eines Phenethanolamins der allgemeinen Formel (I) nach einem der Ansprüche 1 bis 8, dadurch gekennzeichnet, daß man

(a) eine Carbonylverbindung der allgemeinen Formel (II)

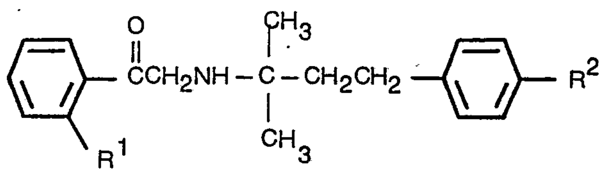

(II)

worin $R^1$ und $R^2$ die in Anspruch 1 angegebenen Bedeutungen haben, reduziert oder

(b) ein Styroloxid der allgemeinen Formel

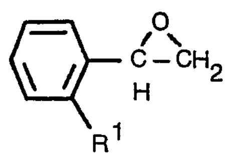

mit einem Amin der allgemeinen Formel

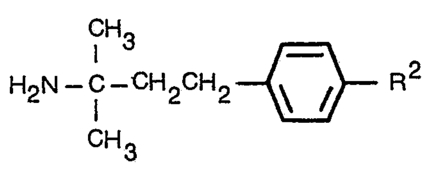

worin $R^1$ und $R^2$ die in Anspruch 1 genannnten Bedeutungen besitzen, umsetzt und

(c) ein nach obiger Reaktion (a) oder (b) erhaltenes Racemat gewünschtenfalls in sein R-Isomer auftrennt.